# EUROPEAN PATENT APPLICATION

(11) **EP 2 522 278 A1**
(43) Date of publication of application: **14.11.2012**
(21) Application number: 12166552.5
(22) Date of filing: 03.05.2012
(51) Int. Cl.: A61B 8/00, A61B 8/14

(54) **Image processing device, image processing method, program, recording medium, image processing system, and probe**

(30) Priority: 13.05.2011 JP 2011108118; 20.01.2012 JP 2012009587
(71) Applicant: Sony Corporation, Tokyo 108-0075 (JP)
(72) Inventor: Kashima, Koji, Minato-ku, Tokyo 108-0075 (JP); Sakaguchi, Tatsumi, Minato-ku, Tokyo 108-0075 (JP); Kajihata, Hiroshi, Minato-ku, Tokyo 108-0075 (JP)
(74) Representative: Keston, Susan Elizabeth

(57) **Abstract**

A sensor information acquisition unit acquires information indicating a position and an orientation of a probe that generates an ultrasonic wave and receives a reflected wave of the ultrasonic wave. A cross-sectional image generation unit generates a cross-sectional image indicating at least a part of a cross-sectional surface of a subject by arranging and synthesizing a plurality of ultrasonic images, which are based on the reflected wave received by the probe at a plurality of positions around the subject, based on the position and the orientation of the probe when the probe generates the ultrasonic wave and receives the reflected wave. The present technique is applicable to, for example, an ultrasonic inspection apparatus.

## Description

The present technique relates to an image processing device, an image processing method, a program, a recording medium, an image processing system, and a probe, and more particularly, to an image processing device, an image processing method, a program, a recording medium, an image processing system, and a probe suitable for generating a cross-sectional image of a subject by the use of ultrasonic waves.

Hitherto, ultrasonic inspection apparatuses have been widely spread in the medical field. In recent years, there have been ultrasonic inspection apparatuses developed and sold as non-medical apparatuses for general purposes by lowering the output power of the ultrasonic waves (for example, see "Development of Healthcare Ultrasonic Echo for Measuring Body Fat and Muscle Mass" by Fukuda and others, Medical Instrument Study, Japanese Society of Medical Instrumentation, Article 78, Part 3, p. 113 to 124, March 1,2008).

In the ultrasonic inspection apparatuses according to the related art, portions touched by a probe are imaged as pinpoints regardless of whether it is for medical or non-medical use. Therefore, for example, the thickness of subcutaneous fat or muscle at the position of the abdominal part touched by the probe can be measured, but it is difficult to view the entire state of the part around the abdominal part at a glance.

On the other hand, the cross-sectional surface of the abdominal part can be visualized when using a medical inspection apparatus using a magnetic resonance imaging (MRI) technique, a computed tomography (CT) technique, or the like. However, since the medical inspection apparatus is large-sized and high-priced or a subject has to lie on a bed to perform inspection, the medical inspection apparatus may not be used simply by a general person.

It is desirable to provide a technique of simply acquiring the cross-sectional image of a subject such as an abdominal part.

Various respective aspects and features of the invention are defined in the appended claims. Combinations of features from the dependent claims may be combined with features of the independent claims as appropriate and not merely as explicitly set out in the claims.

According to a first embodiment of the present technology, there is provided an image processing device including an information acquisition unit that acquires information indicating a position and an orientation of a probe generating an ultrasonic wave and receiving a reflected wave of the ultrasonic wave, and a cross-sectional image generation unit that generates a cross-sectional image indicating at least a part of a cross-sectional surface of a subject by arranging and synthesizing a plurality of ultrasonic images, which are based on the reflected wave received by the probe at a plurality of positions around the subject, based on the position and the orientation of the probe when the probe generates the ultrasonic wave and receives the reflected wave.

The cross-sectional image generation unit may further arrange the ultrasonic images based on information regarding the ultrasonic images.

The cross-sectional image generation unit may detect a motion in a translation direction of the probe based on the change in the orientation of the probe acquired by the information acquisition unit and the information regarding the ultrasonic images and arranges the ultrasonic images based on the orientation of the probe and a motion in the translation direction of the probe.

The cross-sectional image generation unit may match coordinate systems of the ultrasonic images of two frames based on the change in the orientation of the probe, and then detects the motion in the translation direction of the probe based on motions of local feature points between the ultrasonic images of the two frames.

The cross-sectional generation unit may match coordinate systems of the ultrasonic images of two frames based on the change in the orientation of the probe, and then detects the motion in the translation direction of the probe by performing block matching on the ultrasonic images of the two frames.

The information acquisition unit further acquires information regarding a force by which the probe presses against the subject, and based on the force by which the probe presses against the subject and the position and the orientation of the probe, the cross-sectional image generation unit corrects deformation caused when the cross-sectional surface of the subject in the cross-sectional image is pushed by the probe.

When the subject is a living body, the cross-sectional image generation unit may recognize each internal tissue of the living body in the cross-sectional image and correct the deformation caused when each recognized tissue is pushed by the probe.

The cross-sectional image generation unit may calculate a photographing position and a photographing orientation of each of the ultrasonic images based on the position and the orientation of the probe, projects each of the ultrasonic images on a predetermined plane surface, and synthesizes the images projected on the predetermined plane surface based on the photographing position and the photographing orientation of each of the ultrasonic images.

The image processing device may further include a probe state detection unit that detects a state of the probe based on the information acquired by the information acquisition unit. The information acquisition unit may acquire data indicating the position and the orientation of the probe from a plurality of different sensors, and the probe state detection unit selects data to be used to detect the state of the probe among the data acquired by the plurality of sensors.

The image processing device may further include a probe state detection unit that detects a state of the probe based on the information acquired by the information acquisition unit. The information acquisition unit may acquire data indicating the position and the orientation of the probe from a plurality of different sensors, and the probe state detection unit may switch the kinds of sensors to be used to detect the state of the probe based on a setting of a user.

The cross-sectional image generation unit may generate the cross-sectional image by adjusting transmittances of the ultrasonic images and synthesizing the ultrasonic images.

The image processing device may further include an ultrasonic image generation unit that generates the ultrasonic image based on the reflected wave received by the probe.

The image processing device may further include a display control unit that performs control to display an image showing a corresponding pattern among patterns indicating classifications of a cross-sectional state of the subject together with the cross-sectional image or instead of the cross-sectional image.

According to the first embodiment of the present technology, there is provided an image processing method including acquiring information indicating a position and an orientation of a probe generating an ultrasonic wave and receiving a reflected wave of the ultrasonic wave, and generating a cross-sectional image indicating at least a part of a cross-sectional surface of a subject by arranging and synthesizing a plurality of ultrasonic images, which are based on the reflected wave received by the probe at a plurality of positions around the subject, based on the position and the orientation of the probe when the probe generates the ultrasonic wave and receives the reflected wave.

According to the first embodiment of the present technology, there is provided a program for causing a computer to execute a process including acquiring information indicating a position and an orientation of a probe generating an ultrasonic wave and receiving a reflected wave of the ultrasonic wave, and generating a cross-sectional image indicating at least a part of a cross-sectional surface of a subject by arranging and synthesizing a plurality of ultrasonic images, which are based on the reflected wave received by the probe at a plurality of positions around the subject, based on the position and the orientation of the probe when the probe generates the ultrasonic wave and receives the reflected wave.

According to a second embodiment of the present technology, there is provided an image processing system including a probe, and an image processing device. The probe may include an ultrasonic wave generation unit that generates an ultrasonic wave, an ultrasonic wave reception unit that receives a reflected wave of the ultrasonic wave generated by the ultrasonic wave generation unit, and a detection unit that detects a position and an orientation of the probe, and the image processing device include an ultrasonic image generation unit that generates each ultrasonic image based on the reflected wave received by the ultrasonic wave reception unit, and a cross-sectional image generation unit that generates a cross-sectional image indicating at least a part of a cross-sectional surface of a subject by arranging and synthesizing the ultrasonic images at a plurality of positions around the subject based on the position and the orientation of the probe when the probe generates the ultrasonic wave and receives the reflected wave.

According to the second embodiment of the present technology, there is provided an image processing method in an image processing system including a probe and an image processing device, the method including generating, by the probe, an ultrasonic wave, receiving, by the probe, a reflected wave of the generated ultrasonic wave, detecting, by the probe, a position and an orientation of the probe, generating, by the image processing device, each ultrasonic image based on the reflected wave received by the probe, and generating, by the image processing device, a cross-sectional image indicating at least a part of a cross-sectional surface of a subject by arranging and synthesizing the plurality of ultrasonic images at a plurality of positions around the subject based on the position and the orientation of the probe when the probe generates the ultrasonic wave and receives the reflected wave.

According to the third embodiment of the present technology, there is provided a probe including an ultrasonic wave generation unit that generates an ultrasonic wave, an ultrasonic wave reception unit that receives a reflected wave of the ultrasonic wave generated by the ultrasonic wave generation unit, and a detection unit that detects a position and an orientation of the probe to arrange a plurality of ultrasonic images at a plurality of positions around the subject when the plurality of ultrasonic images are synthesized based on the reflected wave received by the ultrasonic wave reception unit.

The detection unit may further detect a force by which the probe presses against the subject.

According to the first embodiment of the present technology, information indicating a position and an orientation of a probe generating an ultrasonic wave and receiving a reflected wave of the ultrasonic wave is acquired, and a cross-sectional image indicating at least a part of a cross-sectional surface of a subject is generated by arranging and synthesizing a plurality of ultrasonic images, which are based on the reflected wave received by the probe at a plurality of positions around the subject, based on the position and the orientation of the probe when the probe generates the ultrasonic wave and receives the reflected wave

According to the second embodiment of the present technology, an ultrasonic wave is generated, a reflected wave of the ultrasonic wave generated is received, a position and an orientation of the probe is detected, and a cross-sectional image is generated a cross-sectional image indicating at least a part of a cross-sectional surface of a subject by arranging and synthesizing the ultrasonic images at a plurality of positions around the subject based on the position and the orientation of the probe when the probe generates the ultrasonic wave and receives the reflected wave.

According to the second embodiment of the present technology, an ultrasonic wave is generated, a reflected wave of the ultrasonic wave generated is received, and a position and an orientation of the probe to arrange a plurality of ultrasonic images at a plurality of positions around the subject when the plurality of ultrasonic images are synthesized based on the reflected wave received by the ultrasonic wave reception unit is detected.

According to the embodiments of the present technique described above, the cross-sectional image of a subject can be obtained simply.

Embodiments of the invention will now be described with reference to the accompanying drawings, throughout which like parts are referred to by like references, and in which:
FIG. 1 is a block diagram illustrating an image processing system according to an embodiment of the present technique;
FIG. 2 is a flowchart illustrating a photographing process;
FIG. 3 is a diagram illustrating an example of the motion of a probe when an abdominal part is photographed;
FIG. 4 is a flowchart illustrating the details of a cross-sectional image generation process;
FIG. 5 is a schematic view illustrating an image in which ultrasonic images are arranged in a virtual space;
FIG. 6 is a diagram illustrating a process of correcting the motion of the probe in upper and lower directions;
FIG. 7 is a diagram illustrating an example of a cross-sectional image;
FIG. 8 is a diagram illustrating examples in which a cross-sectional image is deformed and displayed;
FIG. 9 is a diagram illustrating examples in which a cross-sectional image is deformed and displayed;
FIG. 10 is a block diagram illustrating an image processing system according to a modification example applying the present technique;
FIG. 11 is a block diagram illustrating an example of the functional configuration of a cross-sectional image generation unit;
FIG. 12 is a flowchart illustrating a cross-sectional image generation process according to a modification example;
FIG. 13 is a diagram illustrating a movement direction of the probe;
FIG. 14 is a diagram illustrating a method of detecting a translation vector;
FIG. 15 is a diagram illustrating a first specific example of a method of synthesizing ultrasonic images;
FIG. 16 is a diagram illustrating a second specific example of a method of synthesizing ultrasonic images;
FIG. 17 is a diagram illustrating a third specific example of a method of synthesizing ultrasonic images;
FIG. 18 is a diagram illustrating a method of deforming and synthesizing ultrasonic images;
FIG. 19 is a diagram illustrating a first example of the position of the body of a person to be photographed and the movement direction of the probe;
FIG. 20 is a diagram illustrating a second example of the position of the body of the person to be photographed and the movement direction of the probe; and
FIG. 21 is a block diagram illustrating an example of the configuration of a computer.

Hereinafter, preferred embodiments of the present disclosure will be described in detail with reference to the appended drawings. Note that, in this specification and the appended drawings, structural elements that have substantially the same function and structure are denoted with the same reference numerals, and repeated explanation of these structural elements is omitted.

Hereinafter, a mode (hereinafter referred to as an embodiment) for carrying out the present technique will be described. The description will be made in the following order:

### 1. Embodiment

### 2. Modification Examples.

### 1. Embodiment

### Example of Configuration of Image Processing System 101

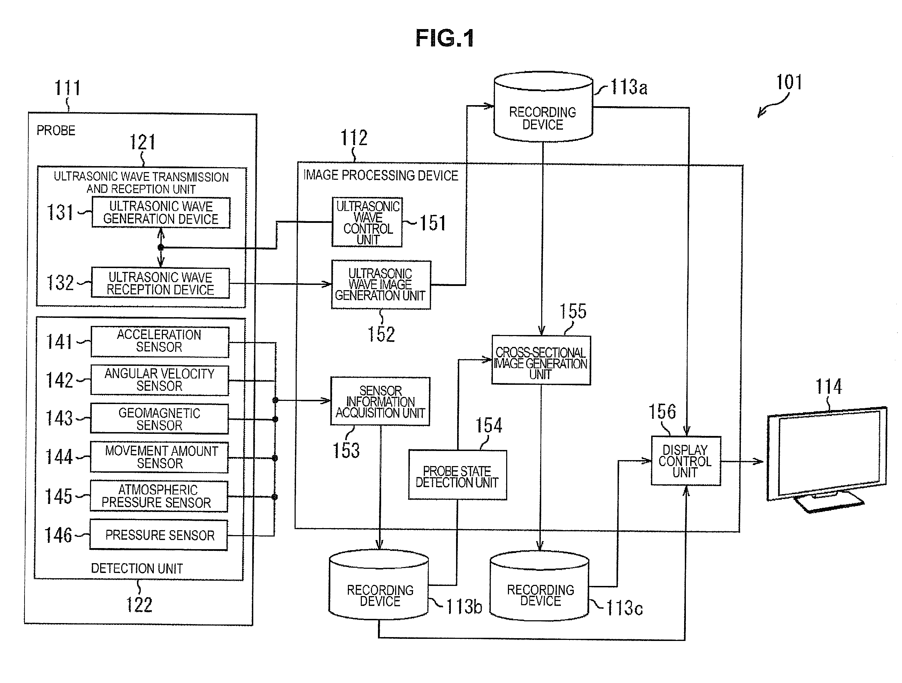

FIG. 1 is a block diagram illustrating an example of the configuration of an image processing system 101 according to an embodiment applying the present technique.

The image processing system 101 is a system that generates and displays a cross-sectional image showing at least a part of the cross-sectional surface of a subject by the use of ultrasonic waves. The image processing system 101 is, for example, an ultrasonic wave inspection apparatus that photographs and inspects a cross-sectional portion of each part of an abdominal part of a human being.

The image processing system 101 includes a probe 111, an image processing device 112, recording devices 113a to 113c, and a display 114.

The probe 111 includes an ultrasonic wave transmission and reception unit 121 and a detection unit 122.

For example, the ultrasonic wave transmission and reception unit 121 is installed in the front end of the probe 111, and transmits and receives ultrasonic waves under the control of an ultrasonic wave control unit 151 of the image processing device 112. The ultrasonic wave transmission and reception unit 121 includes an ultrasonic wave generation device 131 and an ultrasonic wave reception device 132.

The ultrasonic wave generation device 131 generates ultrasonic waves under the control of the ultrasonic wave control unit 151. More specifically, for example, the ultrasonic wave generation device 131 oscillates pulsed ultrasonic waves at a predetermined interval and scans the ultrasonic waves.

Any method of scanning the ultrasonic waves can be used. For example, a method of radially scanning the ultrasonic waves may be used or a method of scanning the ultrasonic waves in parallel may be used. When the ultrasonic waves are scanned radially, a sectorial ultrasonic image can be obtained. When the ultrasonic waves are scanned in parallel, a rectangular ultrasonic image can be obtained.

The ultrasonic wave reception device 132 receives reflected waves of the ultrasonic waves generated by the ultrasonic wave generation device 131 under the control of the ultrasonic wave control unit 151. The ultrasonic wave reception device 132 measures the intensity of the received reflected waves and supplies, for example, data (hereinafter referred to as ultrasonic measured data) indicating the time-series measurement result of the intensity of the reflected waves to an ultrasonic image generation unit 152 of the image processing device 112.

The detection unit 122 detects a state (for example, the position or orientation) of the probe 111. The detection unit 122 includes an acceleration sensor 141, an angular velocity sensor 142, a geomagnetic sensor 143, a movement amount sensor 144, an atmospheric pressure sensor 145, and a pressure sensor 146.

For example, the acceleration sensor 141 detects the acceleration and inclination of the probe 111.

For example, the angular velocity sensor 142 detects a rotational motion (for example, the angular velocity or rotational angle) of the pitch, yaw, and roll of the probe 111 in each direction.

For example, the geomagnetic sensor 143 detects the orientation (azimuth direction) of the probe 111 with respect to the direction of geomagnetism.

For example, the movement amount sensor 144 detects the movement amount of the probe 111 in a translation direction.

For example, the atmospheric pressure sensor 145 detects the position of the probe 111 in a height direction.

For example, the pressure sensor 146 is installed in the vicinity of the installation position of the ultrasonic wave transmission and reception unit 121. The pressure sensor 146 detects a force (hereinafter referred to as a touch pressure) by which the probe 111 presses a subject when the probe 111 touches the subject to photograph its ultrasonic image.

Each sensor of the detection unit 122 supplies sensor data indicating the detection result to a sensor information acquisition unit 153 of the image processing device 112.

The image processing device 112 generates the cross-sectional image of the subject and performs a process of displaying the generated cross-sectional image on the display 114. The image processing device 112 includes the ultrasonic wave control unit 151, the ultrasonic image generation unit 152, the sensor information acquisition unit 153, a probe state detection unit 154, a cross-sectional image generation unit 155, and a display control unit 156.

The ultrasonic wave control unit 151 controls the transmission and reception of the ultrasonic waves of the probe 111 by controlling the ultrasonic wave generation device 131 and the ultrasonic wave reception device 132.

The ultrasonic image generation unit 152 generates an ultrasonic image based on the ultrasonic measured data supplied from the ultrasonic wave reception device 132.

Accordingly, the ultrasonic wave generation device 131, the ultrasonic wave reception device 132, the ultrasonic wave control unit 151, and the ultrasonic image generation unit 152 perform processes of generating the ultrasonic waves, receiving the reflected waves, and generating the ultrasonic image based on the received reflected waves, that is, photograph the ultrasonic image.

The ultrasonic image generation unit 152 stores ultrasonic image data indicating the generated ultrasonic image in the recording device 113a.

The sensor information acquisition unit 153 acquires information indicating the state, such as the position and orientation, of the probe 111. Specifically, the sensor information acquisition unit 153 samples the detection value of each sensor at a predetermined interval based on the sensor data supplied from each sensor of the probe 111. Further, the sensor information acquisition unit 153 stores, as sensor information, the detection value of each sensor and time, at which the detection value is sampled, in the recording device 113b.

The probe state detection unit 154 detects the state of the probe 111 that is photographing an ultrasonic image based on the sensor information stored in the recording device 113b and supplies the detection result to the cross-sectional image generation unit 155.

The cross-sectional image generation unit 155 generates the cross-sectional image of the subject based on the ultrasonic image stored in the recording device 113a and the state of the probe 111 which is photographing the ultrasonic image. The cross-sectional image generation unit 155 stores cross-sectional image data indicating the generated cross-sectional image in the recording device 113c.

The display control unit 156 displays the cross-sectional image of the subject on the display 114 based on the cross-sectional image data stored in the recording device 113c.

### Photographing Process

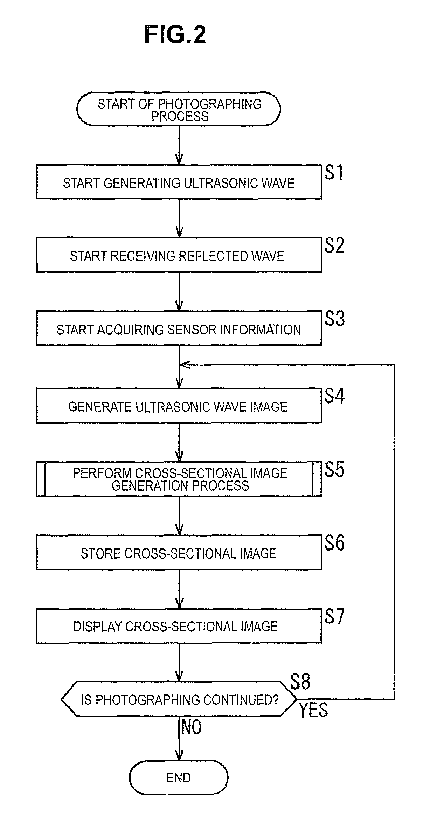

Next, a photographing process performed by the image processing system 101 will be described with reference to the flowchart of FIG. 2.

For example, the photographing process starts when an instruction to start photographing an ultrasonic image is input via an operation unit (not shown) of the image processing system 101.

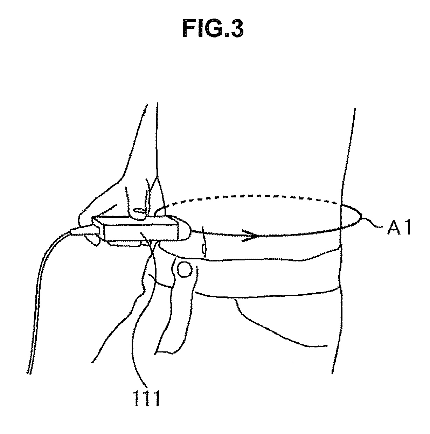

Hereinafter, a case will be described in which the cross-sectional surface of the abdominal part of a human being is photographed using the image processing system 101. In this case, for example, as shown in FIG. 3, a person to be photographed touches and circles the probe 111 substantially vertically to the abdominal part around the abdominal part substantially parallel to the abdominal part, as indicated by an arrow A1, to photograph the cross-sectional surface of the abdominal part.

A person other than the person to be photographed may operate the probe 111 or a remote operation may be performed using a robot arm or the like.

In step S1, the ultrasonic wave generation device 131 starts generating ultrasonic waves under the control of the ultrasonic wave control unit 151. For example, the ultrasonic wave generation device 131 oscillates pulsed ultrasonic waves at a predetermined interval and scans the ultrasonic waves in a predetermined direction.

In step S2, the ultrasonic wave reception device 132 starts receiving the reflected waves of the ultrasonic waves generated by the ultrasonic wave generation device 131 under the control of the ultrasonic wave control unit 151. Then, the ultrasonic wave reception device 132 measures the intensity of the received reflected waves and supplies the ultrasonic measured data indicating the measurement result to the ultrasonic image generation unit 152.

In step S3, the sensor information acquisition unit 153 starts acquiring the sensor information. Specifically, the sensor information acquisition unit 153 samples the detection value of each sensor at a predetermined interval based on the sensor data supplied from each sensor of the probe 111. Then, the sensor information acquisition unit 153 stores, as the sensor information, both the detection value of each sensor and the time at which the detection value is sampled in the recording device 113b.

In step S4, the ultrasonic image generation unit 152 generates the ultrasonic image based on the ultrasonic measured data supplied from the ultrasonic wave reception device 132. That is, the ultrasonic image generation unit 152 generates a two-dimensional ultrasonic image indicating the internal cross-sectional surface in the vicinity of the position at which the abdominal part of the person to be photographed is touched by the probe 111. The ultrasonic image generation unit 152 stores the ultrasonic image data indicating the generated ultrasonic image together with the photographing time in the recording device 113a.

Further, the method of generating the ultrasonic image is not limited, but any method can be utilized.

In step S5, the cross-sectional image generation unit 155 performs a cross-sectional image generation process.

### Details of Cross-sectional Image Generation Process

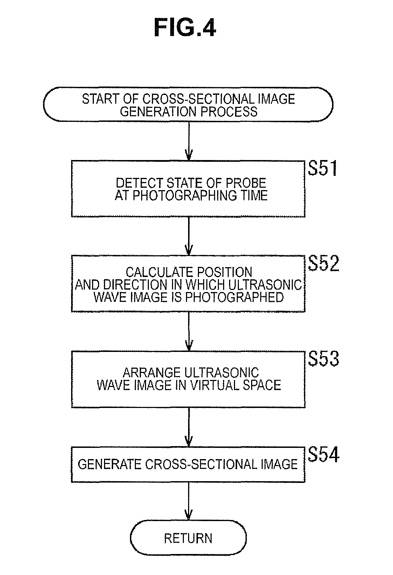

Hereinafter, the cross-sectional image generation process in step S5 will be described in detail with reference to the flowchart of FIG. 4.

In step S51, the probe state detection unit 154 detects the state of the probe 111 at the photographing time based on the sensor information stored in the recording device 113b.

Specifically, the probe state detection unit 154 obtains changes (trajectories) in the position and the orientation of the probe 111 up to the current time based on the detection results of the acceleration sensor 141, the angular velocity sensor 142, the geomagnetic sensor 143, the movement amount sensor 144, and the atmospheric pressure sensor 145.

As described above, the discrete detection value of each sensor is obtained at a predetermined sampling interval. Therefore, the probe state detection unit 154 obtains changes in the position and the orientation of the probe 111 by interpolating the detection value of each sensor, as necessary.

The interpolating method to be used is not limited to a specific method. For example, when it is supposed that the motion of the probe 111 photographing the ultrasonic image is smooth, linear interpolation, spline interpolation, or the like is performed.

Then, the probe state detection unit 154 detects the position and the orientation of the probe 111 based on the changes in the position and the orientation of the probe 111, when the probe 111 generates the ultrasonic waves and receives the reflected waves to photograph the recent ultrasonic image.

The probe state detection unit 154 detects the touch pressure of the probe 111 based on the detection result of the pressure sensor 146, when the probe 111 touches to the abdominal part to photograph the recent ultrasonic image.

Then, the probe state detection unit 154 supplies the detection result of the state of the probe 111 at the photographing time to the cross-sectional image generation unit 155.

In step S52, the cross-sectional image generation unit 155 obtains the position and the orientation in which the ultrasonic image is photographed. Specifically, based on the position and the orientation of the probe 111 at the time of photographing the recent ultrasonic image, the cross-sectional image generation unit 155 calculates the position (photographing position) and the orientation (photographing direction) in which the recent ultrasonic image is photographed. Further, a previous relation between the stereotactic position (the photographing position and photographing direction) of the ultrasonic image and the position and orientation of the probe 111 is used.

In step S53, the cross-sectional image generation unit 155 arranges the ultrasonic images in a virtual space. Specifically, the cross-sectional image generation unit 155 reads the recent ultrasonic images from the recording device 113a. Then, based on the photographing position and the photographing direction of the recent ultrasonic image, the cross-sectional image generation unit 155 arranges the recent ultrasonic images in a three-dimensional virtual space where the ultrasonic images up to the immediately previous frame are arranged. Further, the relative position relation between the ultrasonic images can be calculated based on the photographing position and the photographing direction of each ultrasonic image.

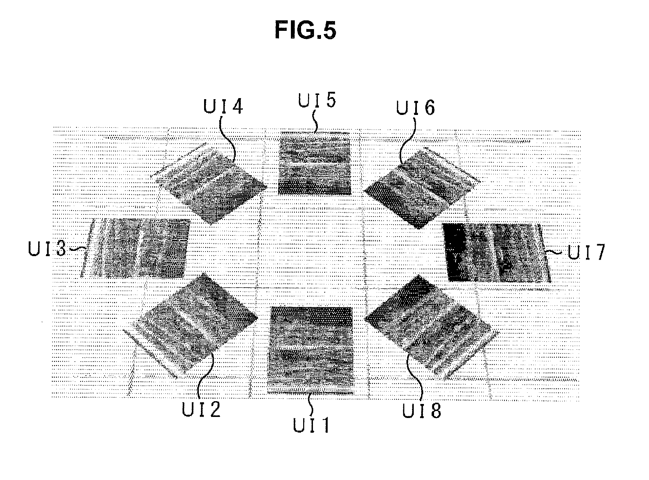

FIG. 5 is a schematic diagram illustrating an image in which ultrasonic images U1 to U18 photographed at a plurality of positions around the abdominal part are arranged in the virtual space.

Based on information regarding the ultrasonic images, the cross-sectional image generation unit 155 adjusts the positions at which the ultrasonic images are arranged.

For example, the cross-sectional image generation unit 155 detects the feature points of the recent ultrasonic images. Then, the cross-sectional image generation unit 155 adjusts the positions at which the ultrasonic images are arranged by tracing the trajectories of the feature points of the ultrasonic images up to the current time.

For example, a sensor generally has a tendency to be weak to the detection of the motion in the translation direction and to increase a detection error. For this reason, when only the sensor information is used, the accuracy of the position adjustment of the ultrasonic images may deteriorate in some cases. Accordingly, when both the sensor information and the information regarding the ultrasonic images are used, the accuracy of the position adjustment of the ultrasonic images is improved.

In contrast, since much noise generally occurs in the ultrasonic images, it is difficult to adjust the positions of the ultrasonic images with high accuracy based on only the information regarding the ultrasonic images. Therefore, when both the sensor information and the information regarding the ultrasonic images are used, the accuracy of the position adjustment of the ultrasonic images is improved.

In step S54, the cross-sectional image generation unit 155 generates the cross-sectional image. That is, the cross-sectional image generation unit 155 generates the two-dimensional cross-sectional image by synthesizing the ultrasonic images arranged in the virtual space.

At this time, in the portion in which the plurality of ultrasonic images overlap, for example, the cross-sectional image generation unit 155 corrects the overlapping images to exactly overlap or selects an image to be used in the overlapping portion.

For example, when the number of ultrasonic images is not abundant and there is a gap between the arranged ultrasonic images, the cross-sectional image generation unit 155 interpolates the images by a predetermined method. Here, the method of interpolating the images is not limited to a specific method.

For example, a template image for the cross-sectional surface of the abdominal part of the standard person may be prepared and the images may be interpolated based on the template image. In this case, for example, a plurality of different template images are preferably prepared for each parameter, such as age, sex, and weight, which has an influence on the shape of the cross-sectional surface of the abdominal part.

Alternatively, for example, the images may be interpolated based on the ultrasonic images obtained previously by photographing the same person to be photographed.

Alternatively, for example, the images may be interpolated by image processing such as in-painting or morphing.

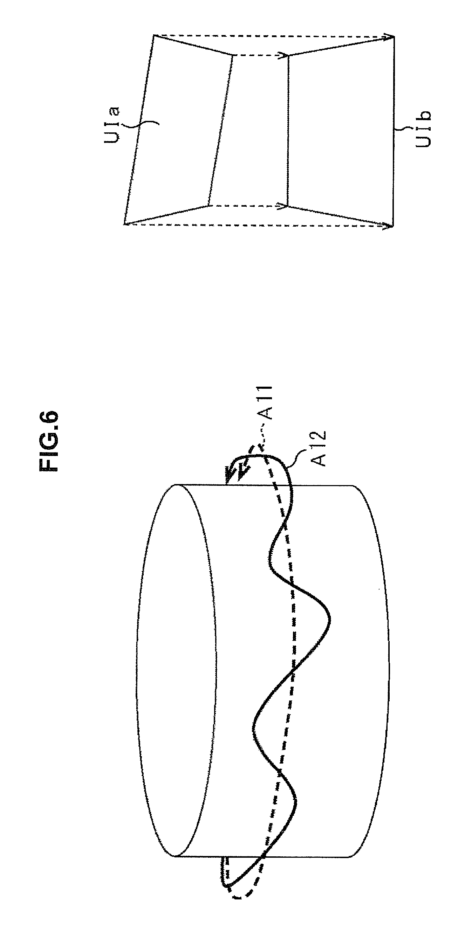

When the probe 111 is moved by hands, it is assumed that the probe 111 is not moved horizontally around the abdominal part, as indicated by an arrow A11 shown in the right side of FIG. 6, but is moved while being vertically waved, as indicated by an arrow A12. In this case, the image obtained by synthesizing the ultrasonic images without interpolation may rarely reproduce the clear cross-sectional surface similar to the well horizontally cut abdominal part.

Accordingly, for example, based on the photographing position and the photographing direction of each ultrasonic image, the cross-sectional image generation unit 155 obtains the inclination of each ultrasonic image with respect to the planar surface (hereinafter referred to as a reference surface) horizontal to the ground surface. For example, the inclination of the probe 111 with respect to the horizontal direction can be detected by the angular velocity sensor 142 or the like. Then, the cross-sectional image generation unit 155 converts each ultrasonic image on the reference surface in a projection manner. For example, as shown in the right side of FIG. 6, the cross-sectional image generation unit 155 generates an ultrasonic image UIb obtained by converting the ultrasonic image Ula on the reference surface in the projection manner so as to eliminate the inclination of an ultrasonic image UIa with respect to the reference surface. Then, the cross-sectional image generation unit 155 synthesizes the ultrasonic images projected on the reference surface.

Thus, the clear cross-sectional surface similar to the horizontally cut abdominal part can be reproduced.

In general, when an ultrasonic image is photographed, an image with a high equality may not be obtained provided that the probe 111 does not closely touch the skin. On the other hand, since the abdominal part is soft, the abdominal part is easily deformed when the probe 111 is tightly pressed thereagainst.

It is supposed that a person examining or inspecting the cross-sectional surface of the abdominal part desires to view the cross-sectional surface of the abdominal part which is not deformed in many cases. Therefore, it is not desirable to show the cross-sectional image of the abdominal part deformed by pushing of the probe 111 without interpolation in some cases.

Accordingly, based on the detection result of the pressure sensor 146, the cross-sectional image generation unit 155 interpolates the deformation of the cross-sectional surface of the abdominal part caused due to the pushing of the probe 111 in the cross-sectional image.

Specifically, the cross-sectional image generation unit 155 first recognizes each internal tissue of the abdominal part in the cross-sectional image. For example, the cross-sectional image generation unit 155 extracts the edge of the generated cross-sectional image and divides the cross-sectional surface of the abdominal part into a plurality of regions based on the extracted edge. Then, based on the texture of each region, the cross-sectional image generation unit 155 recognizes a tissue of the human body corresponding to each region. For example, the cross-sectional image generation unit 155 recognizes a pure white region as bone, a stringy region (a region where a white portion is shown in a streaky shape) as muscle, a marbled region (a region where a white portion is dapped) as fat, etc.

Next, based on the detection result of the changes in the position and the orientation of the probe 111 and the detection result of the touch pressure of the probe 111, the cross-sectional image generation unit 155 obtains the position and orientation of a force (touch pressure) by which the probe 111 presses down on the abdominal part when each ultrasonic image is photographed. Further, based on the previous data such as the dynamic deformation or the position relation between the tissues of the human body, the cross-sectional image generation unit 155 estimates the pressure applied to the tissues of the respective regions and the degree of deformation and dispersion state. Then, the cross-sectional image generation unit 155 estimates the shape of each region in which the pressure of the probe 111 is eliminated.

Finally, the cross-sectional image generation unit 155 generates the cross-sectional image showing the cross-sectional surface of the abdominal part which is not deformed by correcting the shape of each region of the cross-sectional image to the estimated shape.

To further improve the correction accuracy, deformation parameters of the human body of the person to be photographed may be acquired in advance and the correction may be performed by reflecting an individual difference based on the deformation parameters.

In the process of step S54, the cross-sectional image generation unit 155 generates a cross-sectional image showing the intermediate progress up to the range of the photographed ultrasonic images, when the probe 111 is not yet circled around the abdominal part.

### Continuity of Photographing Process

Referring back to FIG. 2, in step S6, the cross-sectional image generation unit 155 stores the cross-sectional image data indicating the generated cross-sectional image in the recording device 113c.

In step S7, the image processing system 101 displays the cross-sectional image. Specifically, the display control unit 156 reads the cross-sectional image data from the recording device 113c. Then, the display control unit 156 displays the cross-sectional image based on the read cross-sectional image data on the display 114.

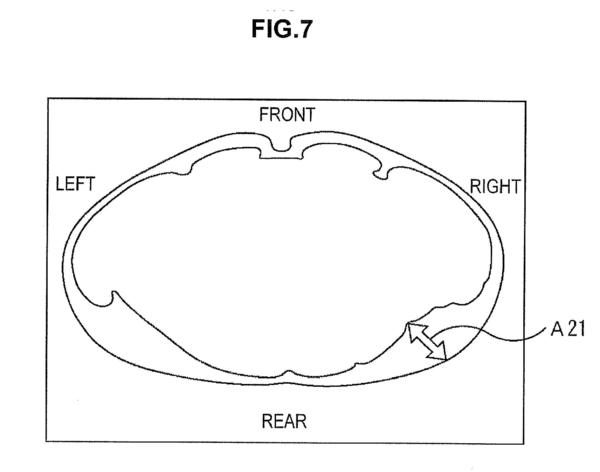

FIG. 7 is a diagram schematically illustrating an example of the displayed cross-sectional image. In FIG. 7, the internal tissues of the abdominal part are not illustrated.

In this example, the cross-sectional image of the abdominal part is displayed directly and the front, rear, right, and left directions are shown. Further, the thickness of the subcutaneous fat is indicated by an arrow A21. Thus, the thickness of the subcutaneous fat is obvious. Further, the numerical value representing the thickness of the subcutaneous fat may be displayed together.

For example, the cross-sectional image may not be displayed directly, but a schematically simplified image (for example, an illustration image) may be displayed.

For example, an image (for example, a deformed image) representing a corresponding pattern among patterns indicating the classification of the cross-sectional state of the abdominal part may be displayed instead of the cross-sectional image.

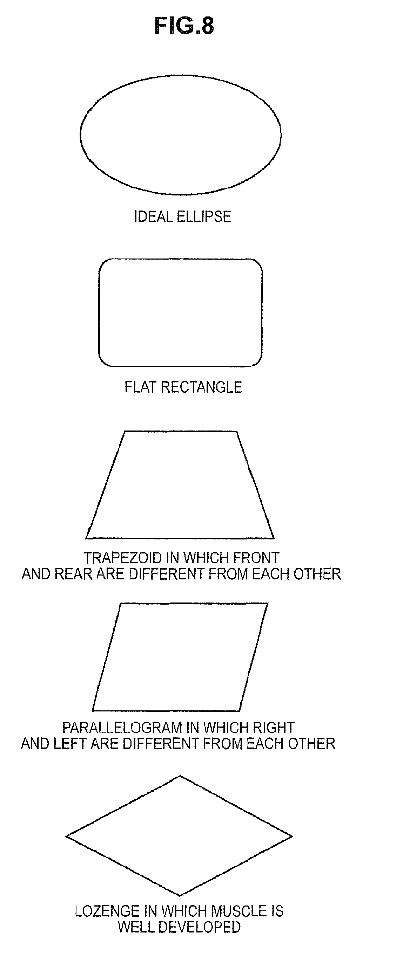

For example, as shown in FIG. 8, the shape of the cross-sectional surface of the abdominal part may be classified into a plurality of patterns and the shape of each pattern may be displayed together with a simple explanation.

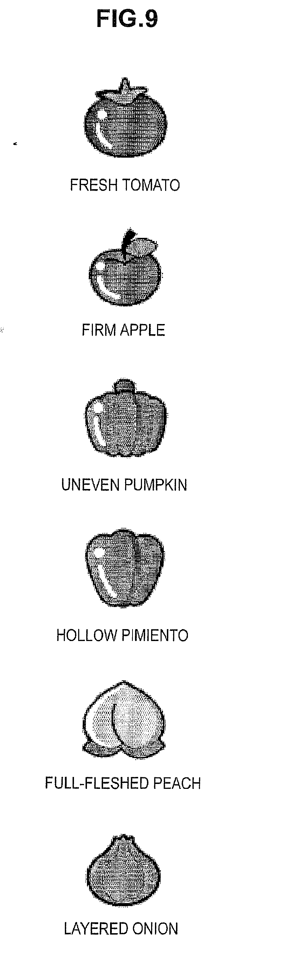

For example, as shown in FIG. 9, the state of the abdominal part may be classified into a plurality of patterns based on the shape of the cross-sectional surface of the abdominal part, the thickness of the subcutaneous fat, or the like and the patterns likened to vegetables or fruits may be displayed as characters together with a simple explanation. Further, animals, anthropomorphic characters, or the like may be used.

The patterns shown in FIG. 8 or 9 may displayed together with the cross-sectional image.

In step S8, the image processing system 101 determines whether the photographing process continues. When the image processing system 101 determines that the photographing process continues, the process returns to step S4.

Thereafter, the processes of steps S4 to S8 are repeatedly performed until it is determined that the photographing process does not continue in step S8. That is, the ultrasonic images are photographed, the cross-sectional image is generated, and the displaying process is continuously performed.

The interval at which the ultrasonic images are photographed and the cross-sectional image is generated is determined in consideration of the processing capability of the image processing system, the capacities of the recording devices 113a to 113c, or the like. Further, when the image processing system 101 is driven by a battery, the interval may be determined in consideration of the capacity of the battery.

For example, when the quality of the recent ultrasonic image is poor due to the fact that the probe 111 is not correctly touched to the abdominal part, the processes of steps S5 to S7 may not be performed, and the cross-sectional image may be not generated and displayed.

On the other hand, in step S8, for example, when an instruction to terminate the photographing process is input via the operation unit (not shown) of the image processing system 101, the image processing system 101 determines that the photographing process does not continue and terminates the photographing process.

Thus, whether a person has received special training or has no specialized medical knowledge, the cross-sectional image of the abdominal part can be simply acquired by a simple operation.

Since the position adjustment of the ultrasonic images is performed and the ultrasonic images are synthesized based on both the information regarding the ultrasonic images and the sensor information, the cross-sectional surface of the abdominal part can be reproduced with high accuracy.

Since the probe 111 may be simply moved around the abdominal part, for example, the cross-sectional image of the abdominal part can be photographed at any position of a human body such as an erect position, a sitting position, or a supine position.

When it is not necessary to circle the probe 111 around the abdominal part and the probe 111 is not circled, the cross-sectional image showing a part of the cross-sectional surface of the abdominal part can be generated.

### 2. Modification Examples

Hereinafter, modification examples of the embodiment of the present technique will be described.

### Modification Example 1: Modification Example of Application Target of Present Technique

The example has hitherto been described in which the abdominal part of a human being is photographed. However, for example, the cross-sectional surface of a head part, cervical part, a chest part, the limbs, or the like can, of course, be photographed according to an embodiment of the present technique. Further, a subject to be inspected using a cross-sectional image is not particularly limited. For example, not only subcutaneous fat and muscle but also internal organs, bones, tendons, and blood vessels can be inspected.

The present technique can be applicable to both medical purposes and non-medical purposes. When the present technique is applied to non-medical purposes, for example, the frequency and the intensity of the ultrasonic wave frequency are preferably adjusted properly so that internal organs are not shown.

The present technique is applicable to not only human beings but also animals, plants, artificial objects, or the like to photograph various cross-sectional surfaces of a subject by the ultrasonic waves. Modification Example 2: Modification Example of Generation Timing of Cross-sectional Image.

The example has hitherto been described in which the cross-sectional image is generated and displayed in real time while the ultrasonic images are photographed. However, all of the ultrasonic images may first be photographed, and then the cross-sectional image may be generated and displayed.

When the cross-sectional image is configured to be generated and displayed in real time, for example, a user can adjust the movement speed or position of the probe 111 while the user actually confirms the images. Therefore, a more preferred cross-sectional image can be acquired.

### Modification Example 3: Modification Example of Photographing of Ultrasonic Images and Acquisition Timing of Sensor Information

The photographing of the ultrasonic images and the acquisition of the sensor information may or may not be synchronized. When the photographing of the ultrasonic images and the acquisition of the sensor information are not synchronized, the times at which the ultrasonic images are photographed and the time at which the sensor information is acquired are preferably recorded so that the correspondent relation can be known later.

### Modification Example 4: Modification Example of Kinds of Sensors

The kinds of sensors of the probe 111 described above are merely examples. The kinds of sensors may be added or may not be used or different kinds of sensors may be used, as necessary. For example, when it is not necessary to correct the deformation of the cross-sectional surface of a subject, the pressure sensor 146 may not be used.

### Modification Example 5: Example in which Tomographic Image is Generated

A three-dimensional image may be generated by generating a plurality of cross-sectional images while shifting the probe 111 in the circulation direction of the probe 111 and the vertical direction thereto and by synthesizing the cross-sectional images. For example, the cross-sectional image of an abdominal part can be generated by generating cross-sectional images at a plurality of positions in the vertical direction to the abdominal part and synthesizing the cross-sectional images.

### Modification Example 6: Example in which Re-Photographing is Suggested

When suitable ultrasonic images cannot be synthesized based on the sensor information, for example, if the subject being photographed moves more than expected, the subject may be suggested to be re-photographed.

### Modification Example 7: Modification Example of Information Used to Generate Ultrasonic Images

In an embodiment of the present technique, a cross-sectional image may be generated, for example, by synthesizing the ultrasonic images using only the sensor information without use of the information regarding the ultrasonic images.

### Modification Example 8: Modification Example of Configuration of Image Processing System

For example, the arrangement of the respective units of the image processing system 101 is not limited to the example shown in FIG. 1, but may be arbitrarily modified. For example, the probe 111 and the image processing device 112 may be integrated with each other. For example, the ultrasonic wave control unit 151 and the ultrasonic image generation unit 152 may be included in the probe 111. Further, for example, the recording devices 113a to 113c may be integrated with each other or may be mounted on the image processing device 112.

### Modification Example 9: Modification Example of Communication Method or the Like between Units

The respective units may communicate with each other by wired communication or wireless communication.

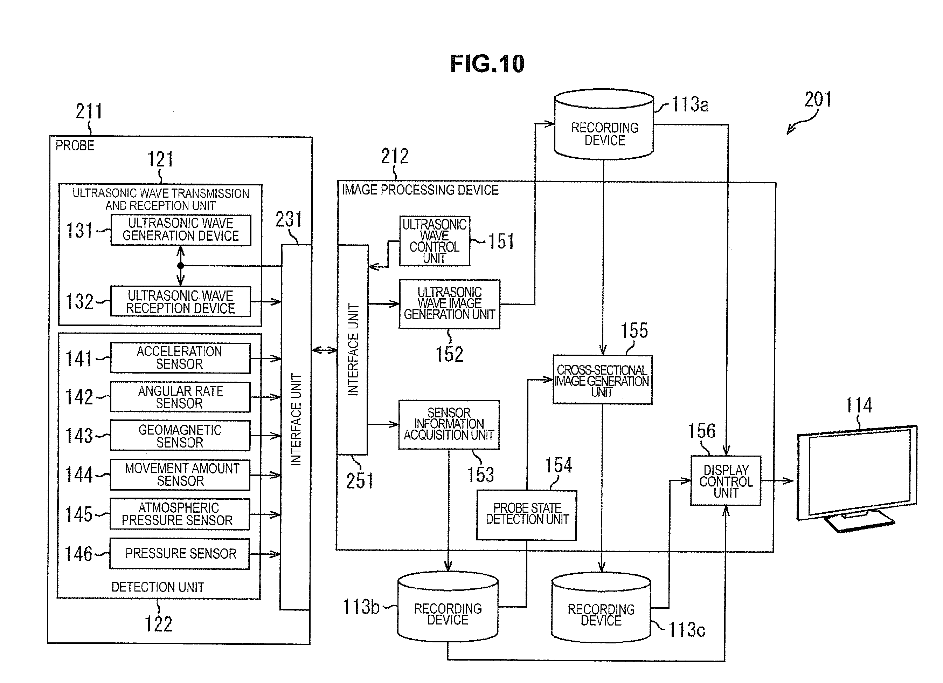

FIG. 10 is a block diagram illustrating an example of the configuration of an image processing system 201 configured so that a probe 211 and an image processing device 212 communicate with each other wirelessly. The same reference numerals are given to the units corresponding to the units in FIG. 1, and the description thereof will appropriately not be repeated.

The image processing system 201 is different from the image processing system 101 in FIG. 1 in that the probe 211 and the image processing device 212 are installed instead of the probe 111 and the image processing device 112. The probe 211 is different from the probe 111 in that an interface unit 231 is further added. The image processing device 212 is different from the image processing device 112 in that an interface unit 251 is further added.

The interface unit 231 of the probe 211 and the interface unit 251 of the image processing device 212 are configured by, for example, communication devices that communicate wirelessly according to a predetermined scheme.

Accordingly, the ultrasonic measured data and the sensor data of each sensor are transmitted from the probe 211 to the image processing device 212 by the wireless communication. Further, the control of the ultrasonic wave generation device 131 and the ultrasonic wave reception device 132 by the image processing device 212 is realized by the wireless communication.

Thus, since a cable or the like connecting the probe 211 to the image processing device 212 is not necessary, the probe 211 can be more easily moved. As a result, the operability of the probe 211 is improved. Thus, for example, a desired position of a body can be photographed more simply.

For example, the ultrasonic measured data and the sensor data may be recorded in a removable medium and may be input to the image processing device 112 via the removable medium. Modification Example 10: Modification Example of Method of Generating Cross-Sectional Image

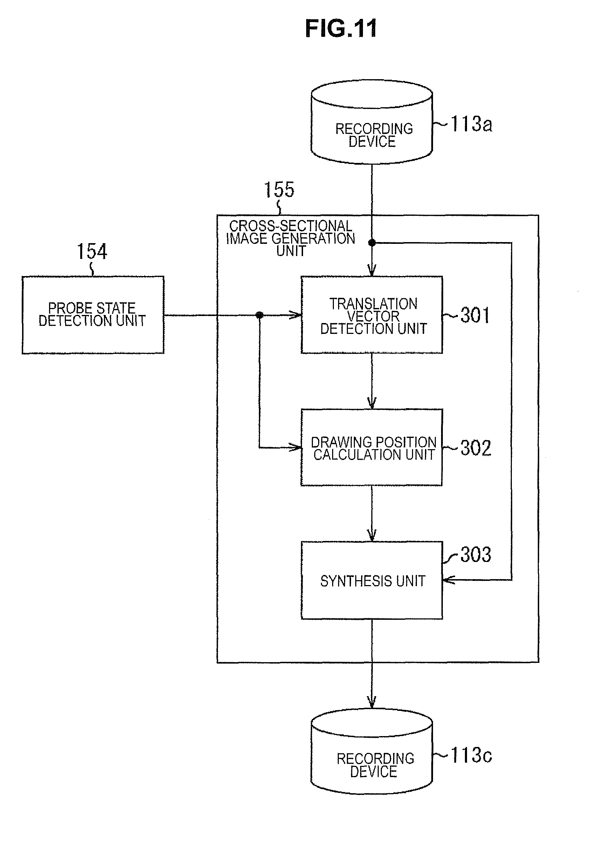

The cross-sectional image generation process described above with reference to FIG. 4 is merely an example, and another method may be used. Here, a modification example of the cross-sectional image generation process will be described in detail with reference to FIGS. 11 to 18.

FIG. 11 is a block diagram illustrating an example of the functional configuration of the cross-sectional image generation unit 155 of the image processing device 112 in FIG. 1.

The cross-sectional image generation unit 155 includes a translation vector detection unit 301, a drawing position calculation unit 302, and a synthesis unit 303.

The translation vector detection unit 301 detects a motion in the translation direction of the probe 111 based on the ultrasonic images stored in the recording device 113a and the state of the probe 111 detected by the probe state detection unit 154. The translation- vector detection unit 301 supplies the detection result of the motion in the translation direction of the probe 111 to the drawing position calculation unit 302.

The drawing position calculation unit 302 calculates the drawing positions of the ultrasonic images based on the state of the probe 111 detected by the probe state detection unit 154 and the motion in the translation direction of the probe 111 detected by the translation vector detection unit 301. The drawing position calculation unit 302 supplies the calculated drawing positions of the ultrasonic images to the synthesis unit 303.

The synthesis unit 303 generates the cross-sectional image of a subject by synthesizing the ultrasonic images stored in the recording device 113a based on the drawing positions calculated by the drawing position calculation unit 302. The synthesis unit 303 stores cross-sectional image data indicating the generated cross-sectional image in the recording device 113c.

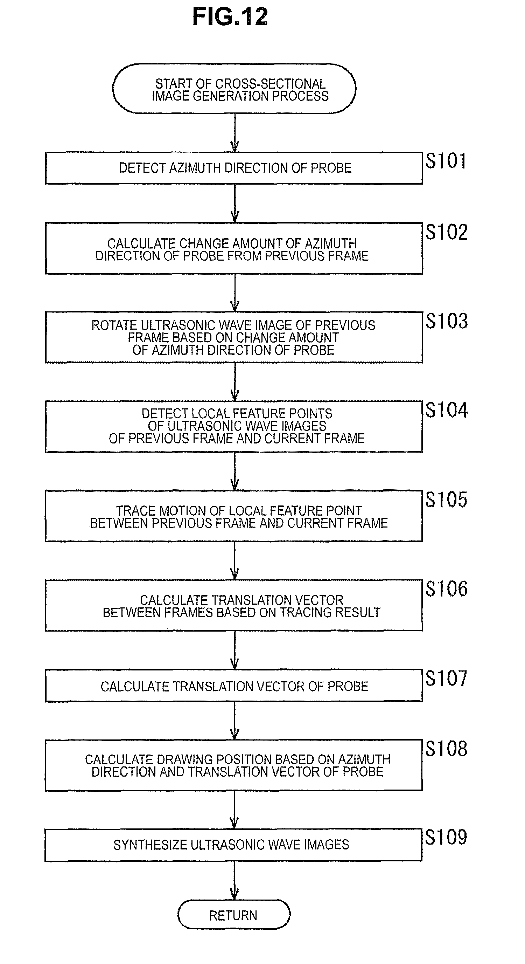

Next, a modification example of the cross-sectional image generation process of step S5 in FIG. 2 will be described in detail with reference to the flowchart of FIG. 12.

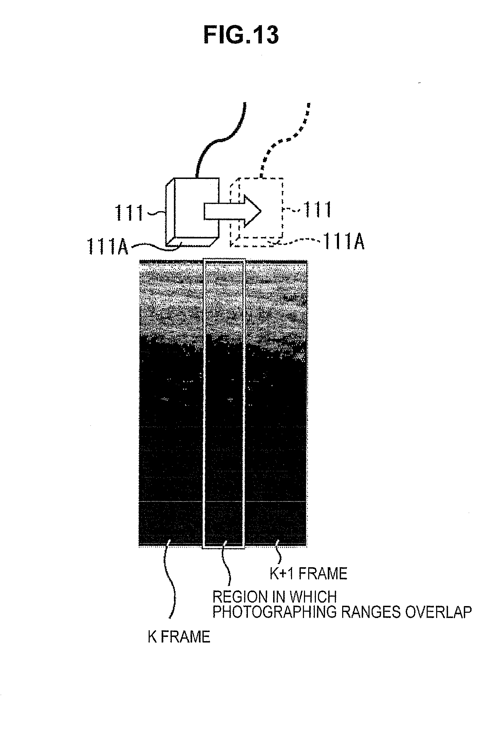

To facilitate the following description, a case will be described in which the ultrasonic images are photographed by vertically touching the probe 111 to the abdominal part, as shown in FIG. 3 described above, moving the probe 111 in the long-side direction of a touch surface 111A, as shown in FIG. 13, and circling the probe 111 horizontally around the abdominal part. Further, the probe 111 is assumed to be a one-dimensional array probe in which an oscillator is arranged one-dimensionally in the long-side direction of the touch surface. Furthermore, it is assumed that the ultrasonic images are photographed so that the photographing ranges of the adjacent frames overlap, as shown in FIG. 13, by setting the movement speed at a sufficiently fast frame rate or moving the probe 111 slowly.

In step S 101, the probe state detection unit 154 detects the azimuth direction of the probe 111 at the photographing time, that is, the orientation of the probe 111 with respect to the direction of the geomagnetism based on the detection result of the geomagnetic sensor 143 stored in the recording device 113b.

In step S102, the probe state detection unit 154 calculates the change amount of the azimuth direction of the probe 111 from the previous frame based on the detection result of the azimuth direction of the probe 111.

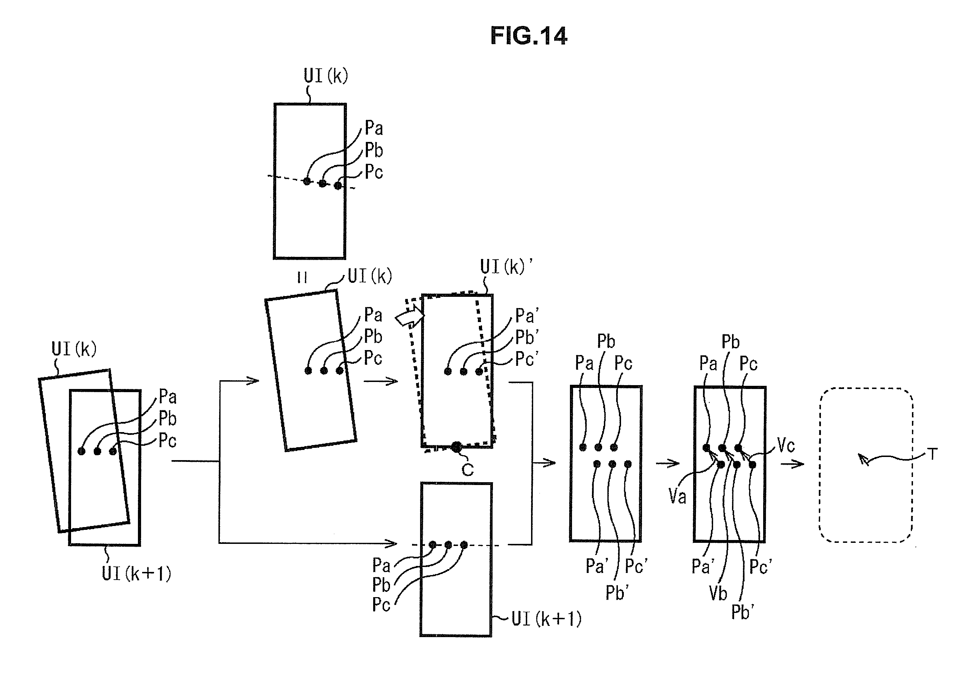

Hereinafter, a case will be described in which the change amount of the azimuth direction between an ultrasonic image UI(k+1) of the recent k+1-th frame and an ultrasonic image UI(k) of the immediately previous k-th frame is calculated with reference to FIG. 14. For example, on the assumption that θa is an azimuth direction detected by the geomagnetic sensor 143 when the ultrasonic image UI(k) is photographed and θb is an azimuth direction detected by the geomagnetic sensor 143 when the ultrasonic image UI(k+1) is photographed, "Δθ=θb-θa" is calculated as the change amount of the azimuth direction of the probe 111. Here, Δθ represents a motion in the rotation direction of the horizontal direction (yaw direction) of the probe 111 from the photographing time of the ultrasonic image UI(k) to the photographing time of the ultrasonic image UI(k+1).

There is a concern that an error may occur in the azimuth direction detected by the geomagnetic sensor 143 due to the influence of the surrounding magnetic field or the like, but the change amount of the azimuth direction is rarely affected by the surrounding magnetic field or the like. Accordingly, by calculating the change amount Δθ of the azimuth direction, it is possible to accurately detect the motion in the rotation direction of the horizontal direction of the probe 111.

The probe state detection unit 154 supplies information regarding the calculated change amount of the azimuth direction of the probe 111 to the translation vector detection unit 301.

In step S103, the translation vector detection unit 301 rotates the ultrasonic image of the previous frame based on the change amount of the azimuth direction of the probe 111.

For example, as shown in FIG. 14, the ultrasonic image UI(k) is rotated by only the change amount Δθ in the azimuth direction of the probe 111 about a rotation center C by a two-dimensional affine transform. Thus, the coordinate system of an ultrasonic image UI(k)' (hereinafter referred to as a rotated image UI(k)') after the rotation matches the coordinate system of the ultrasonic image UI(k+1).

In step S104, the translation vector detection unit 301 detects local feature points of the ultrasonic images of the previous and current frames. More specifically, the translation vector detection unit 301 detects the local feature points of the rotated image of the previous frame and the local feature points of the ultrasonic image of the current frame.

Further, any kind of local feature point and any detection method can be utilized. For example, the Harris Corner Detector, which is resilient against the deformation of a subject to be photographed and suitable for soft human tissues, is used as the local feature point.

In step S105, the translation vector detection unit 301 traces the motion of the local feature points between the previous and current frames. More specifically, the translation vector detection unit 301 traces the local feature points of the rotated image of the previous frame and the local feature points of the ultrasonic image of the current frame.

Any method can be used as the method of tracing the motion of the local feature points. For example, an optical flow Lucas-Kanade method, which is resilient against the deformation of a subject to be photographed and suitable for soft human tissues, is used.

For example, as shown in FIG. 14, when the same local feature points P 1 to P3 are shown in both of the ultrasonic images UI(k) and UI(k+1), local feature points P1' to P3' corresponding to the local feature points P1 to P3 are detected from a rotated image UI(k)'. Further, the local feature points P1 to P3 are detected from the ultrasonic image UI(k+1). Then, a vector Va oriented from a local feature point Pa' to a local feature point Pa is detected, a vector Vb oriented from a local feature point Pb' to a local feature point Pb is detected, and a vector Vc oriented from a local frame point Pc' to a local feature point Pc is detected.

In step S106, the translation vector detection unit 301 calculates a translation vector between the frames based on the trace result. That is, based on the vectors Va to Vc, the translation vector detection unit 301 calculates a translation vector T between the ultrasonic images UI(k) and UI(k+1) by any method. For example, the translation vector detection unit 301 selects one of the vectors Va to Vc as the translation vector T or calculates an average vector of the vectors Va to Vc as the translation vector T.

In step S107, the translation vector detection unit 301 calculates the translation vector of the probe 111. Specifically, the translation vector detection unit 301 calculates an inverse vector inverted from the translation vector T calculated in the process of step S 106 as the translation vector of the probe 111. The translation vector expresses the motion of the probe 111 in the translation direction from the photographing time of the ultrasonic image UI(k) to the photographing time of the ultrasonic image UI(k+1). The translation vector detection unit 301 supplies information indicating the calculated translation vector of the probe 111 to the drawing position calculation unit 302.

In step S108, the drawing position calculation unit 302 calculates the drawing position based on the azimuth direction and the translation vector of the probe 111. Specifically, the drawing position calculation unit 302 calculates the drawing position of the ultrasonic image of the current frame in the three-dimensional virtual space, where the ultrasonic images up to the immediately previous frame are arranged, based on the azimuth direction of the probe 111 detected by the geomagnetic sensor 143 and the translation vector of the probe 111 detected by the translation vector detection unit 301.

For example, the drawing position calculation unit 302 calculates the relative change amount of drawing position between the ultrasonic images of the immediately previous frame and the current frame based on the azimuth direction and the translation vector of the probe 111. Then, the drawing position calculation unit 302 calculates the drawing position of the ultrasonic image of the current frame in the virtual space based on the calculated change amount. The drawing position calculation unit 302 supplies information indicating the calculated drawing position of the ultrasonic image of the current frame to the synthesis unit 303.

In step S109, the synthesis unit 303 synthesizes the ultrasonic images. That is, the synthesis unit 303 generates the cross-sectional image by arranging the ultrasonic image of the current frame at the drawing position calculated by the drawing position calculation unit 302 and synthesizing the ultrasonic images up to the current frame.

Then, the cross-sectional image generation process is ended.

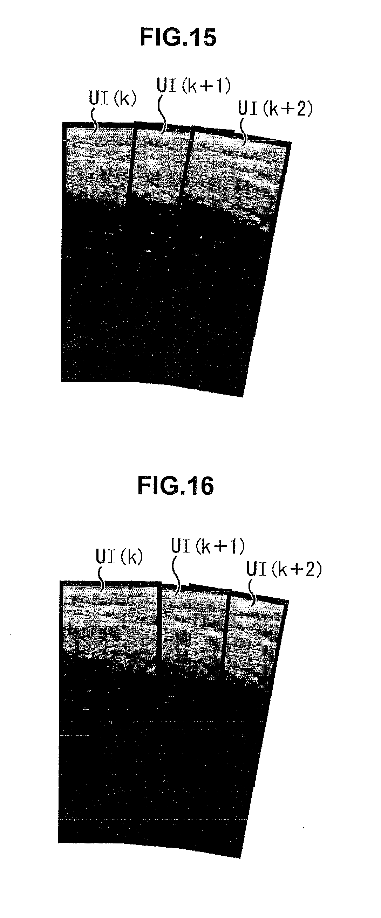

Hereinafter, a specific example of the method of synthesizing the ultrasonic images will be described with reference to FIGS. 15 to 17.

For example, as shown in FIG. 15, simply writing a new ultrasonic image over an old ultrasonic image may be considered. In this case, the portions in which the plurality of ultrasonic images overlap are updated gradually by the new ultrasonic image.

Further, for example, as shown in FIG. 16, adding only a portion of the newly added ultrasonic image which is not shown in the ultrasonic images up to the immediately previous frame may be considered. This case is equivalent to a case in which a new ultrasonic image is simply written over an old ultrasonic image.

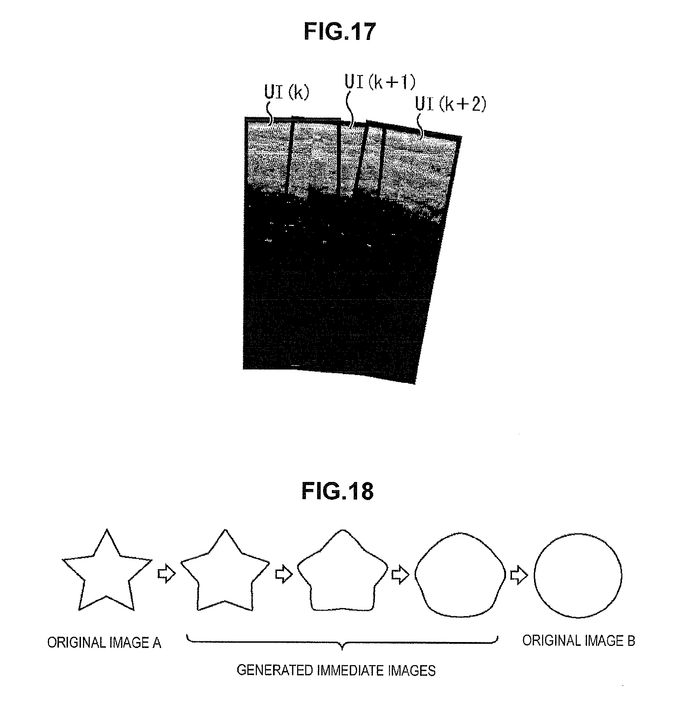

Furthermore, for example, as shown in FIG. 17, synthesizing portions in which the plurality of ultrasonic images overlap in a weighted manner by setting the transmittances of the ultrasonic images and synthesizing the ultrasonic images may be considered. The transmittance of each ultrasonic image may be fixed or may be adjusted dynamically in each frame.

When the transmittance is adjusted dynamically in each frame, for example, setting the transmittance based on the evaluation criterion such as the degree of importance or the image quality of a portion showing the ultrasonic image may be considered. For example, setting the transmittance of the ultrasonic image in which an important part such as an organ is shown to be low and setting the ratio at which the components of the corresponding ultrasonic image are synthesized to be high may be considered. Further, for example, setting the transmittance of an ultrasonic image with a poor image quality, such as an ultrasonic image with a high blur amount or an ultrasonic image with a large correction amount due to projection conversion or the like, to be low and setting the ratio at which the components of the corresponding ultrasonic image are synthesized to be low may be considered. Thus, even when a deviation occurs in the estimated drawing position, the roughness of the generated cross-sectional image can be obscured.

For example, the example shown in FIG. 15 can be realized by setting the transmittance of each ultrasonic image to 0% and writing a new ultrasonic image over an old ultrasonic image. The example shown in FIG. 16 can be realized, for example, by setting the transmittance of a portion of a newly added ultrasonic image shown in the ultrasonic images up to the immediately previous frame to 100%, setting a portion which is not shown to 0%, and writing the new ultrasonic image, as well as adding (writing) only a portion of a newly added ultrasonic image which is not shown in the ultrasonic images up to the immediately previous frame, as described above. Further, the example shown in FIG. 16 can be realized, for example, by setting the transmittance of each ultrasonic image to 0% and writing an old ultrasonic image over a new ultrasonic image.

As described above, the abdominal part of a human being is soft and easily deformed. Therefore, to reduce a sense of discomfort of a synthesized portion, among the images in the portion in which the plurality of ultrasonic images overlap, deforming a deformed image by image processing such as morphing, and then performing synthesis may be considered. For example, as shown in FIG. 18, synthesizing intermediate images generated during a return process of returning a deformed original image A to an original image B before the deformation by morphing may be considered. Accordingly, even when a deviation occurs in the estimated drawing position, the roughness of the generated cross-sectional image can be obscured.

As described above with reference to FIG. 6, the ultrasonic images can be synthesized after each ultrasonic image is projected on the reference surface based on the inclination of the probe 111 detected by the angular velocity sensor 142 or the like.

The example has hitherto been described in which the translation vector is calculated using the local feature points of the ultrasonic image. However, the translation vector may be calculated by another method. For example, a global motion vector between frames may be calculated as the translation vector by motion estimation using block matching. This method is particularly effective when a subject to be photographed is not considerably deformed. That is, the motion estimation using block matching is used as a standard method in moving-image compression. Therefore, abundant processors such as central processing units (CPUs) or graphics processing units (GPUs) provide this function. Accordingly, the motion estimation can be said to be excellent in terms of cost or performance such as a processing speed.

As described above, the shape of an abdominal part is easily deformed. Therefore, when block matching is performed, the accuracy of the block matching can be improved by deforming blocks based on the detection result of the pressure sensor 146 and then performing a matching process.

The example has hitherto been described in which the ultrasonic image of the immediately previous frame is rotated. However, the coordinate systems of two ultrasonic images may be made to match each other by rotating the ultrasonic image of the current frame.

The translation vector may be calculated using two ultrasonic images distant from each other by two or more frames, as well as the ultrasonic images of the adjacent (continuous) frames.

The example has hitherto been described in which only the rotation of the probe 111 in the horizontal direction (yaw direction) is considered. However, even when the probe 111 is rotated in a pitch direction or a roll direction, the drawing position of the ultrasonic image can be calculated by the same method.

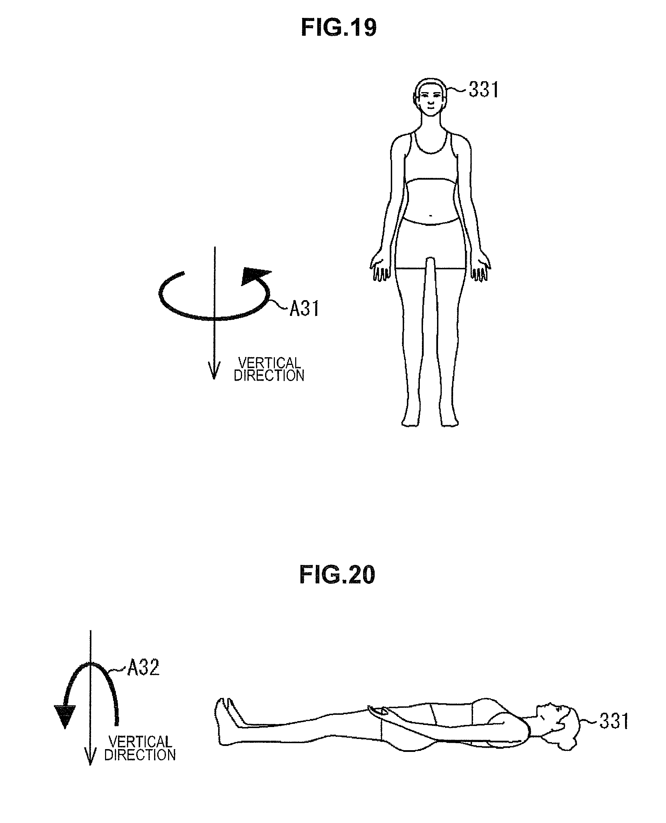

In the image processing system 101, as described above, the cross-sectional image of an abdominal part can be photographed at any position of a human body. However, the movement direction of the probe 111 is different depending on the position of the body of a subject to be photographed. For example, as shown in FIG. 19, it is supposed that when a person 331 to be photographed is photographed at an erected state, the probe 111 is rotated around the abdominal part of the person 331 to be photographed, as indicated by an arrow A31, on a planar surface parallel (perpendicular to a vertical direction) to the ground surface. As shown in FIG. 20, it is supposed that when the person 331 to be photographed is photographed at a lying state, the probe 111 is rotated around the abdominal part of the person 331 to be photographed, as indicated by an arrow A3 2, on a planar surface perpendicular (parallel to a vertical direction) to the ground surface.

On the other hand, the geomagnetic sensor 143 detects the orientation of the probe 111 with respect to the direction of geomagnetism. Therefore, when the probe 111 is horizontally rotated, as shown in FIG. 19, the change amount of orientation of the probe 111 can be detected with high accuracy. However, when the probe 111 is vertically rotated, as shown in FIG. 20, the detection accuracy of the change amount of orientation of the probe 111 may deteriorate.

Accordingly, when the probe 111 is vertically rotated, as shown in FIG. 20, for example, the change amount of orientation of the probe 111 may be detected by the angular velocity sensor 142 instead of the geomagnetic sensor 143.

The sensor to be used may be switched manually or automatically.

When the sensor is switched manually, for example, photographing modes such as an erect position (or a sitting position) photographing mode and a supine position photographing mode may be provided, and the photographing mode may be selected by a user. When the erect position photographing mode is set, the geomagnetic sensor 143 may be used. When the supine position photographing mode is set, the angular velocity sensor 142 may be used.

On the other hand, for example, when all of the ultrasonic images are first photographed and the cross-sectional image is then generated, the sensor to be used may be switched automatically. For example, when both sensor data of the angular velocity sensor 142 and the geomagnetic sensor 143 during the photographing of the ultrasonic images are stored in the recording device 113b, the sensor data to be used can be selected based on a predetermined condition at the time of generating the cross-sectional image. For example, the reliabilities of the sensor data may be calculated and the sensor data to be used may be selected based on the reliabilities. For example, the sensor data to be used may be selected based on the movement direction of the probe 111 detected by another sensor or the position of the body of the person 331 to be photographed.

When a plurality of different sensors can be likewise substituted with each other, the probe state detection unit 154 may switch the sensor used to detect the state of the probe 111 based on a predetermined condition.

### Configuration Example of Computer

The above-described series of processing may be performed by hardware or may be performed by software. When the series of processing is performed by software, a program forming the software is installed into a computer that is incorporated in a dedicated hardware, or installed from a program storage medium into a general-purpose personal computer, for example, that can perform various types of functions by installing various types of programs.

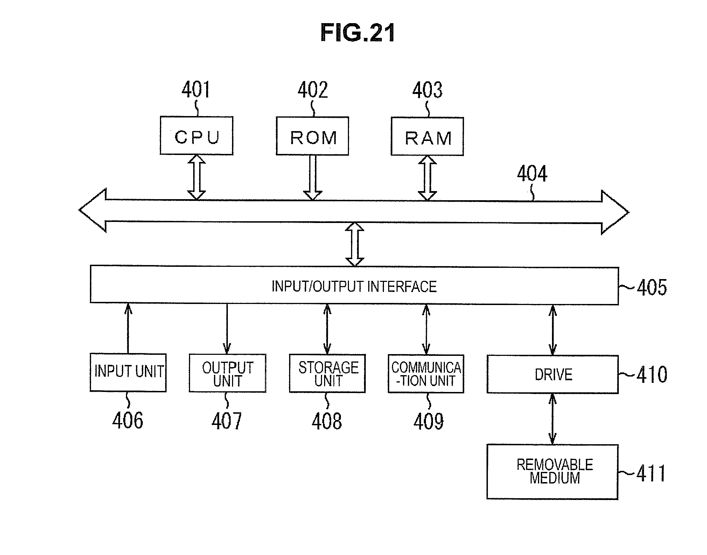

FIG. 21 is a block diagram showing a hardware configuration example of a computer that performs the above-described series of processing using a program.

In the computer, a central processing unit (CPU) 401, a read only memory (ROM) 402 and a random access memory (RAM) 403 are mutually connected by a bus 404.

Further, an input/output interface 405 is connected to the bus 404. Connected to the input/output interface 405 are an input unit 406, an output unit 407, a storage unit 408, a communication unit 409, and a drive 410.

The input unit 406 is configured from a keyboard, a mouse, a microphone or the like. The output unit 407 configured from a display, a speaker or the like. The storage unit 408 is configured from a hard disk, a non-volatile memory or the like. The communication unit 409 is configured from a network interface or the like. The drive 410 drives a removable media 411 such as a magnetic disk, an optical disk, a magneto-optical disk, a semiconductor memory or the like.

In the computer configured as described above, the CPU 401 loads a program that is stored, for example, in the storage unit 408 onto the RAM 403 via the input/output interface 405 and the bus 404, and executes the program. Thus, the above-described series of processing is performed.

Programs to be executed by the computer (the CPU 401) are provided being recorded in the removable media 411 which is a packaged media or the like. Also, programs may be provided via a wired or wireless transmission medium, such as a local area network, the Internet or digital satellite broadcasting.

Then, by inserting the removable media 411 into the drive 410, the program can be installed in the storage unit 408 via the input/output interface 405. Further, the program can be received by the communication unit 409 via a wired or wireless transmission media and installed in the storage unit 408. Moreover, the program can be installed in advance in the ROM 402 or the storage unit 408.

It should be noted that the program executed by a computer may be a program that is processed in time series according to the sequence described in this specification or a program that is processed in parallel or at necessary timing such as upon calling.

In the specification, the terminology "system" means a general device including a plurality of devices and units. That is, in the specification, the system refers to a collection of a plurality of constituent elements (devices, modules (components), or the like), and all of the constituent elements may not be present in the same casing. Accordingly, the system is either the plurality of devices accommodated in separate casings and connected to each other via a network or a single device accommodating the plurality of modules in a single casing.

Embodiments of the present technique are not limited to the above-described embodiments, but may be modified in various forms without departing from the gist of the present technique.

For example, according to an embodiment of the present technique, cloud computing can be realized so that one function is distributed to a plurality of devices via a network so that the function can be processed cooperatively.

The steps described above in the flowcharts may be performed by a single device or may be performed cooperatively by a plurality of devices.

When one step includes a plurality of processes, the plurality of processes of the one step may be performed by a single device or may be performed cooperatively by a plurality of devices.

Additionally, the present technology may also be configured according to the numbered clauses as below.
(1) An image processing device including:
   an information acquisition unit that acquires information indicating a position and an orientation of a probe generating an ultrasonic wave and receiving a reflected wave of the ultrasonic wave; and
   a cross-sectional image generation unit that generates a cross-sectional image indicating at least a part of a cross-sectional surface of a subject by arranging and synthesizing a plurality of ultrasonic images, which are based on the reflected wave received by the probe at a plurality of positions around the subject, based on the position and the orientation of the probe when the probe generates the ultrasonic wave and receives the reflected wave.
(2) The image processing device according to (1), wherein the cross-sectional image generation unit further arranges the ultrasonic images based on information regarding the ultrasonic images.
(3) The image processing device according to (2), wherein the cross-sectional image generation unit detects a motion in a translation direction of the probe based on the change in the orientation of the probe acquired by the information acquisition unit and the information regarding the ultrasonic images and arranges the ultrasonic images based on the orientation of the probe and a motion in the translation direction of the probe.
(4) The image processing device according to (3), wherein the cross-sectional image generation unit matches coordinate systems of the ultrasonic images of two frames based on the change in the orientation of the probe, and then detects the motion in the translation direction of the probe based on motions of local feature points between the ultrasonic images of the two frames.
(5) The image processing device according to (3), wherein the cross-sectional generation unit matches coordinate systems of the ultrasonic images of two frames based on the change in the orientation of the probe, and then detects the motion in the translation direction of the probe by performing block matching on the ultrasonic images of the two frames.
(6) The image processing device according to any one of (1) to (5),
   wherein the information acquisition unit further acquires information regarding a force by which the probe presses against the subject, and
   based on the force by which the probe presses against the subject and the position and the orientation of the probe, the cross-sectional image generation unit corrects deformation caused when the cross-sectional surface of the subject in the cross-sectional image is pushed by the probe.
(7) The image processing device according to (6), wherein when the subject is a living body, the cross-sectional image generation unit recognizes each internal tissue of the living body in the cross-sectional image and corrects the deformation caused when each recognized tissue is pushed by the probe.
(8) The image processing device according to any one of (1) to (7), wherein the cross-sectional image generation unit calculates a photographing position and a photographing orientation of each of the ultrasonic images based on the position and the orientation of the probe, projects each of the ultrasonic images on a predetermined plane surface, and synthesizes the images projected on the predetermined plane surface based on the photographing position and the photographing orientation of each of the ultrasonic images.
(9) The image processing device according to any one of (1) to (8), further comprising:
   a probe state detection unit that detects a state of the probe based on the information acquired by the information acquisition unit,
   wherein the information acquisition unit acquires data indicating the position and the orientation of the probe from a plurality of different sensors, and
   the probe state detection unit selects data to be used to detect the state of the probe among the data acquired by the plurality of sensors.
(10) The image processing device according to any one of (1) to (8), further including:
   a probe state detection unit that detects a state of the probe based on the information acquired by the information acquisition unit,
   wherein the information acquisition unit acquires data indicating the position and the orientation of the probe from a plurality of different sensors, and
   the probe state detection unit switches the kinds of sensors to be used to detect the state of the probe based on a setting of a user.
(11) The image processing device according to any one of (1) to (10), wherein the cross-sectional image generation unit generates the cross-sectional image by adjusting transmittances of the ultrasonic images and synthesizing the ultrasonic images.
(12) The image processing device according to any one of (1) to (11), further including:
   an ultrasonic image generation unit that generates the ultrasonic image based on the reflected wave received by the probe.
(13) The image processing device according to any one of (1) to (12), further including:
   a display control unit that performs control to display an image showing a corresponding pattern among patterns indicating classifications of a cross-sectional state of the subject together with the cross-sectional image or instead of the cross-sectional image.
(14) An image processing method including:
   acquiring information indicating a position and an orientation of a probe generating an ultrasonic wave and receiving a reflected wave of the ultrasonic wave; and
   generating a cross-sectional image indicating at least a part of a cross-sectional surface of a subject by arranging and synthesizing a plurality of ultrasonic images, which are based on the reflected wave received by the probe at a plurality of positions around the subject, based on the position and the orientation of the probe when the probe generates the ultrasonic wave and receives the reflected wave.
(15) A program for causing a computer to execute a process including:
   acquiring information indicating a position and an orientation of a probe generating an ultrasonic wave and receiving a reflected wave of the ultrasonic wave; and
   generating a cross-sectional image indicating at least a part of a cross-sectional surface of a subject by arranging and synthesizing a plurality of ultrasonic images, which are based on the reflected wave received by the probe at a plurality of positions around the subject, based on the position and the orientation of the probe when the probe generates the ultrasonic wave and receives the reflected wave.
(16) A computer-readable recording medium recording the program according to (15).
(17) An image processing system including:
   a probe; and
   an image processing device,
   wherein the probe includes
      an ultrasonic wave generation unit that generates an ultrasonic wave,
      an ultrasonic wave reception unit that receives a reflected wave of the ultrasonic wave generated by the ultrasonic wave generation unit, and
      a detection unit that detects a position and an orientation of the probe, and
   the image processing device includes,
      an ultrasonic image generation unit that generates each ultrasonic image based on the reflected wave received by the ultrasonic wave reception unit, and
      a cross-sectional image generation unit that generates a cross-sectional image indicating at least a part of a cross-sectional surface of a subject by arranging and synthesizing the ultrasonic images at a plurality of positions around the subject based on the position and the orientation of the probe when the probe generates the ultrasonic wave and receives the reflected wave.
(18) An image processing method in an image processing system including a probe and an image processing device, the method including:
   generating, by the probe, an ultrasonic wave;
   receiving, by the probe, a reflected wave of the generated ultrasonic wave;
   detecting, by the probe, a position and an orientation of the probe;
   generating, by the image processing device, each ultrasonic image based on the reflected wave received by the probe; and
   generating, by the image processing device, a cross-sectional image indicating at least a part of a cross-sectional surface of a subject by arranging and synthesizing the plurality of ultrasonic images at a plurality of positions around the subject based on the position and the orientation of the probe when the probe generates the ultrasonic wave and receives the reflected wave.
(19) A probe including:
   an ultrasonic wave generation unit that generates an ultrasonic wave;
   an ultrasonic wave reception unit that receives a reflected wave of the ultrasonic wave generated by the ultrasonic wave generation unit; and
   a detection unit that detects a position and an orientation of the probe to arrange a plurality of ultrasonic images at a plurality of positions around the subject when the plurality of ultrasonic images are synthesized based on the reflected wave received by the ultrasonic wave reception unit.
(20) The probe according to claim 19, wherein the detection unit further detects a force by which the probe presses against the subject.

It should be understood by those skilled in the art that various modifications, combinations, subcombinations and alterations may occur depending on design requirements and other factors insofar as they are within the scope of the appended claims or the equivalents thereof.

In so far as the embodiments of the invention described above are implemented, at least in part, using software-controlled data processing apparatus, it will be appreciated that a computer program providing such software control and a transmission, storage or other medium by which such a computer program is provided are envisaged as aspects of the present invention.

## Claims

1. An image processing device comprising:
an information acquisition unit that acquires information indicating a position and an orientation of a probe generating an ultrasonic wave and receiving a reflected wave of the ultrasonic wave; and
a cross-sectional image generation unit that generates a cross-sectional image indicating at least a part of a cross-sectional surface of a subject by arranging and synthesizing a plurality of ultrasonic images, which are based on the reflected wave received by the probe at a plurality of positions around the subject, based on the position and the orientation of the probe when the probe generates the ultrasonic wave and receives the reflected wave.

2. The image processing device according to claim 1, wherein the cross-sectional image generation unit detects a motion in a translation direction of the probe based on the change in the orientation of the probe acquired by the information acquisition unit and information regarding the ultrasonic images and arranges the ultrasonic images based on the orientation of the probe and a motion in the translation direction of the probe.

3. The image processing device according to claim2, wherein the cross-sectional image generation unit matches coordinate systems of the ultrasonic images of two frames based on the change in the orientation of the probe, and then detects the motion in the translation direction of the probe based on motions of local feature points between the ultrasonic images of the two frames.

4. The image processing device according to claim2, wherein the cross-sectional generation unit matches coordinate systems of the ultrasonic images of two frames based on the change in the orientation of the probe, and then detects the motion in the translation direction of the probe by performing block matching on the ultrasonic images of the two frames.

5. The image processing device according to claim 1,
wherein the information acquisition unit further acquires information regarding a force by which the probe presses against the subject, and
based on the force by which the probe presses against the subject and the position and the orientation of the probe, the cross-sectional image generation unit corrects deformation caused when the cross-sectional surface of the subject in the cross-sectional image is pushed by the probe.

6. The image processing device according to claim 5, wherein when the subject is a living body, the cross-sectional image generation unit recognizes each internal tissue of the living body in the cross-sectional image and corrects the deformation caused when each recognized tissue is pushed by the probe.

7. The image processing device according to claim 1, wherein the cross-sectional image generation unit calculates a photographing position and a photographing orientation of each of the ultrasonic images based on the position and the orientation of the probe, projects each of the ultrasonic images on a predetermined plane surface, and synthesizes the images projected on the predetermined plane surface based on the photographing position and the photographing orientation of each of the ultrasonic images.

8. The image processing device according to claim 1, further comprising:
a probe state detection unit that detects a state of the probe based on the information acquired by the information acquisition unit,
wherein the information acquisition unit acquires data indicating the position and the orientation of the probe from a plurality of different sensors, and
the probe state detection unit selects data to be used to detect the state of the probe among the data acquired by the plurality of sensors.

9. The image processing device according to claim 1, further comprising:
a probe state detection unit that detects a state of the probe based on the information acquired by the information acquisition unit,
wherein the information acquisition unit acquires data indicating the position and the orientation of the probe from a plurality of different sensors, and
the probe state detection unit switches the kinds of sensors to be used to detect the state of the probe based on a setting of a user.

10. An image processing method comprising:
acquiring information indicating a position and an orientation of a probe generating an ultrasonic wave and receiving a reflected wave of the ultrasonic wave; and
generating a cross-sectional image indicating at least a part of a cross-sectional surface of a subject by arranging and synthesizing a plurality of ultrasonic images, which are based on the reflected wave received by the probe at a plurality of positions around the subject, based on the position and the orientation of the probe when the probe generates the ultrasonic wave and receives the reflected wave.

11. A program for causing a computer to execute a process including:
acquiring information indicating a position and an orientation of a probe generating an ultrasonic wave and receiving a reflected wave of the ultrasonic wave; and
generating a cross-sectional image indicating at least a part of a cross-sectional surface of a subject by arranging and synthesizing a plurality of ultrasonic images, which are based on the reflected wave received by the probe at a plurality of positions around the subject, based on the position and the orientation of the probe when the probe generates the ultrasonic wave and receives the reflected wave.

12. A computer-readable recording medium recording the program according to claim 11.

13. An image processing system comprising:
a probe; and
an image processing device,
wherein the probe includes
an ultrasonic wave generation unit that generates an ultrasonic wave,
an ultrasonic wave reception unit that receives a reflected wave of the ultrasonic wave generated by the ultrasonic wave generation unit, and
a detection unit that detects a position and an orientation of the probe, and
the image processing device includes,
an ultrasonic image generation unit that generates each ultrasonic image based on the reflected wave received by the ultrasonic wave reception unit, and
a cross-sectional image generation unit that generates a cross-sectional image indicating at least a part of a cross-sectional surface of a subject by arranging and synthesizing the ultrasonic images at a plurality of positions around the subject based on the position and the orientation of the probe when the probe generates the ultrasonic wave and receives the reflected wave.

14. An image processing method in an image processing system including a probe and an image processing device, the method comprising:
generating, by the probe, an ultrasonic wave;
receiving, by the probe, a reflected wave of the generated ultrasonic wave;
detecting, by the probe, a position and an orientation of the probe;
generating, by the image processing device, each ultrasonic image based on the reflected wave received by the probe; and
generating, by the image processing device, a cross-sectional image indicating at least a part of a cross-sectional surface of a subject by arranging and synthesizing the plurality of ultrasonic images at a plurality of positions around the subject based on the position and the orientation of the probe when the probe generates the ultrasonic wave and receives the reflected wave.

15. A probe comprising:
an ultrasonic wave generation unit that generates an ultrasonic wave;
an ultrasonic wave reception unit that receives a reflected wave of the ultrasonic wave generated by the ultrasonic wave generation unit; and
a detection unit that detects a position and an orientation of the probe to arrange a plurality of ultrasonic images at a plurality of positions around the subject when the plurality of ultrasonic images are synthesized based on the reflected wave received by the ultrasonic wave reception unit.
